Europäisches Patentamt

European Patent Office

Office européen des brevets

(19)

(11) Publication number: **0 045 222**
**B1**

(12) **EUROPEAN PATENT SPECIFICATION**

(45) Date of publication of patent specification: **29.10.86**

(21) Application number: **81303498.0**

(22) Date of filing: **30.07.81**

(51) Int. Cl.⁴: **C 12 N 9/02,** A 61 K 37/50 // C12R1/425

(54) **Mn-Superoxide dismutase, its production and use.**

(30) Priority: **30.07.80 JP 105645/80**

(43) Date of publication of application:
**03.02.82 Bulletin 82/05**

(45) Publication of the grant of the patent:
**29.10.86 Bulletin 86/44**

(84) Designated Contracting States:
**AT BE CH DE FR GB IT LI LU NL SE**

(56) References cited:
**EP-A-0 070 656**

**JOURNAL OF GENERAL AND APPLIED MICROBIOLOGY, vol. 22, no. 3, 1976 TOKYO (JP) A.A. YOUSTEN et al.: "A single form of superoxide dismutase found in bacillus popilliae and in some other gram-positive bacteria" pages 161-164**

The file contains technical information submitted after the application was filed and not included in this specification

(73) Proprietor: **Takeda Chemical Industries, Ltd.
27, Doshomachi 2-chome Higashi-ku
Osaka-shi Osaka, 541 (JP)**

(72) Inventor: **Kouichi, Miyata
6-77 Seiwadainishi 1-chome
Kawanishi Hyogo 666-01 (JP)**
Inventor: **Kazutaka, Maejima
8-6 Daiwanishi 5-chome
Kawanishi Hyogo 666-01 (JP)**
Inventor: **Katsumi, Tomoda
17-2 Sakasedai 5-chome
Takarazuka Hyogo 665 (JP)**

(74) Representative: **Laredo, Jack Joseph et al
Elkington and Fife High Holborn House 52/54
High Holborn
London, WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(58) References cited:

CURRENT MICROBIOLOGY, vol. 1, no. 6, 1978
NEW YORK (US) S.S. BANG et al.:
"Electrophoretic mobilities of superoxide
dismutases from species of photobacterium,
beneckea, vibrio and selected terrestrial
enterobacteria" pages 371-376

JOURNAL OF BACTERIOLOGY, vol. 134 no. 1,
April 1978 BALTIMORE, Md (US) L. BRITTON et
al.: "Superoxide dismutase and oxygen
metabolism in streptococcus faecalis and
comparisons with other organisms" pages
229-236

LA RECHERCHE, vol. 10, no. 106, December
1979 PARIS (FR) A.M. MICHELSON: "Une
enzyme qui nous veut du bien" pages 1269-
1270

# 0 045 222

**Description**

This invention relates to Mn-superoxide dismutase, its production and use.

The present inventors undertook a diligent research for discovering a new Mn-superoxide dismutase and found that a microorganism belonging to the genus *Serratia* was able to produce such a new Mn-superoxide dismutase. This finding was followed by a further series of studies which have resulted in the present invention described in detail hereinafter.

This invention is directed to: (1) a Mn-superoxide dismutase having a defined set of properties, (2) a method for producing said Mn-superoxide dismutase characterised by cultivating a Mn-superoxide dismutase-producing microorganism belonging to the genus *Serratia* in a culture medium to thereby cause said microorganism to elaborate and accumulate said Mn-superoxide dismutase in said culture and harvesting said Mn-superoxide dismutase from the resulting culture broth, and (3) an antiinflammatory agent which contains an effective amount of said Mn-superoxide dismutase having a defined set of properties as an active ingredient in association with a pharmaceutically acceptable carrier or excipient therefor.

The microorganism employed for production of the contemplated substance of this invention is any of the microorganisms belonging to the genus *Serratia* and capable of producing Mn-superoxide dismutase.

Among such microorganisms there are mentioned *Serratia liquefaciens, Serratia marcescens, Serratia mironorubra*, and concretely mentioned *Serratia liquefaciens* IFO 12979, *Serratia marcescens* IFO 3046, *Serratia marcescens* IFO 3759 (ATCC 4002), *Serratia marcescens* IFO 3736, *Serratia marinorubra* IFO 12973 (ATCC 27614).

The above microorganisms designated by IFO numbers are listed up in the Institute for Fermentation Osaka List of Cultures 1978 Sixth Edition, published by Institute for Fermentation, Osaka, Japan, and the microorganisms designated by ATCC numbers are listed up in the American Type Culture Collection Catalogue of Strains I Fourteenth Edition 1980 published by American Type Culture Collection, Rockville, Maryland, U.S.A.

The production of the contemplated superoxide dismutase is carried out by cultivating said microorganisms in a culture medium and harvesting and purifying the resulting elaboration product, which is the desired product, from said culture broth.

The culture medium may be liquid or solid, as long as it contains nutrient sources which the particular strain of microorganism can utilize. A liquid culture medium is desirable when mass production is contemplated.

Incorporated in such a medium are sources of nutrients which the microorganism can assimilate, digest or otherwise utilize, such as carbon sources, nitrogen sources, inorganic materials, trace nutrients and so on. The carbon sources may for example be starch, dextrin, glucose, sucrose, etc., and the nitrogen sources may for example be corn steep liquor, soybean meal, extracted soybean meal, yeast extract, denucleated yeast, casein, cotton seed meal, nitrogen-containing compounds such as ammonium chloride, ammonium sulfate, etc. The inorganic materials include, for example, sodium salts (e.g. sodium chloride), phosphates (e.g. sodium phosphate), calcium salts (e.g. calcium carbonate), potassium salts (e.g. potassium chloride, potassium phosphate), magnesium salts (e.g. magnesium sulfate), manganese salts (e.g. manganese chloride, manganese carbonate), iron salts (e.g. iron chloride), zinc salts (e.g. zinc carbonate) and so on. The trace nutrients may for example be vitamins (e.g. vitamin $B_1$, $B_2$), fatty acid esters [e.g. esters (e.g. methyl or ethyl esters) of oleic acid, lauric acid, caproic acid, etc.)], nucleic acids (e.g. ribonucleic acid, deoxyribonucleic acid), related compounds of nucleic acids (e.g. inosinic acid, guanylic acid, adenylic acid) and so on. For defoaming purposes, oils (e.g. soybean oil, corn oil, peanut oil), synthetic antifoams [e.g. Tween 20, 60 and 80 (Kao-Atlas Co. Ltd., Japan), Actocol® (Takeda Chemical Industries, Ltd., Japan)], etc. may also be incorporated.

The cultivation method may for example be stationary culture, shake culture or aerated stir (submerged aerobic) culture. Submerged aerobic culture, in particular, is especially useful for production-scale runs. While cultural conditions vary with the condition and composition of medium, strain of microorganism, cultural method, etc., cultivation is desirably conducted at about 20 to 40°C for about 10 to 100 hours, preferably, about 20 to 72 hours, the pH of the medium being about 4 to 9 and, preferably, about 6 to 8.

The desired enzyme is mostly accumulated intracellularly. This enzyme is harvested and purified by conventional purification procedures. It is advantageous to collect the cells from the culture broth by centrifugation, filtration or some other known separatory method, disrupt the cells by such a procedure as ultrasonication, grinding with glass beads, or a method involving the use of a surfactant or solvent, and extract the enzyme from the disrupted mass. In harvesting the enzyme, the same or another known separation and purification procedure in the field of the art can be followed in any case to obtain a product of optional purity. Thus, the enzyme-containing disrupted mass is centrifuged or filtered to give a supernatant or filtrate. To this enzyme extract is added ammonium sulfate or acetone, and the resulting precipitate is collected by centrifugation, dissolved in a small quantity of water, desalted by dialysis and lyophilized, whereupon a crude enzyme powder is obtained. This crude enzyme powder can be purified by means employed conventionally for purification of enzymes. By way of illustration, gel filtration with Sephadex® (Pharmacia Fine Chemicals AB, Sweden), column chromatography on diethylaminoethyl-

3

0 045 222

cellulose (Serva Fine Biochimica, West Germany) or a like procedure can be followed to isolate superoxide dismutase. In this manner, superoxide dismutase can be separated and isolated in a desired purity.

The enzymological and chemical properties of the superoxide dismutase obtained in Example 2 which appears hereinafter are as follows.

(a) Activity: It dismutates a superoxide ion into a hydrogen peroxide molecule and an oxygen molecule.

(b) Substrate specificity: It acts on superoxide ion.

(c) Optimum pH range: The optimum pH range of this enzyme is about 7 to 8.

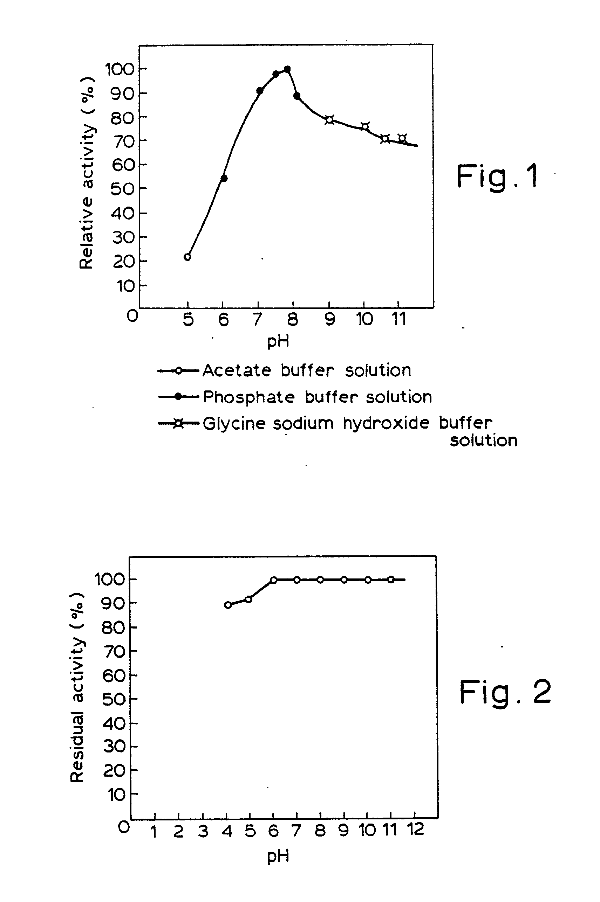

Fig. 1 shows a pH-activity curve of this enzyme.

(d) Stable pH range: The enzyme is stable in the pH range of about 6 to 11.

Fig. 2 shows a pH-stability curve of this enzyme.

(e) Optimum functional temperature: About 20 to 45°C.

(f) Thermostability (conditions of inactivation): The enzyme was treated at pH 7.8 and various temperatures for various time periods. The enzyme is stable at 37°C for 60 minutes but is inactivated about 50% when treated at 50 to 60°C for 60 minutes and almost 100% when treated at 80°C for 5 minutes.

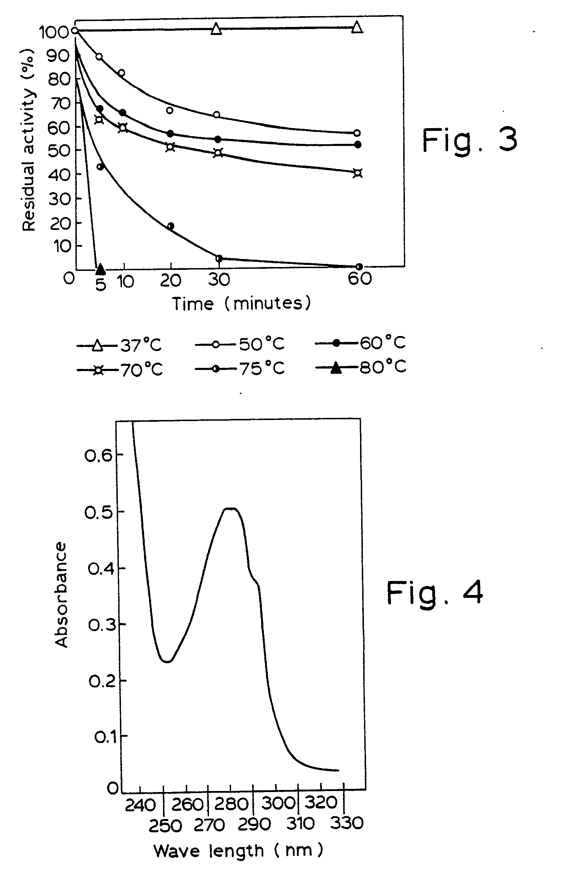

Fig. 3 shows a thermostability of this enzyme.

(g) Molecular weight: As determined by the gel filtration method using a Sephadex G-100® column [The Biochemical Journal *91*, 222—233, 1964], this enzyme has a molecular weight of about $4.8 \times 10^4$. When the enzyme is first treated with 1% sodium lauryl-sulfate (SDS) and then subjected to polyacrylamide gel electrophoresis in the presence of the SDS [The Journal of Biological Chemistry *244*, 5074—5080, 1969], the enzyme is found to have a molecular weight of about $2.4 \times 10^4$. These values indicate that this enzyme is a dimer consisting of subunits each having a molecular weight of about $2.4 \times 10^4$.

(h) Elemental analysis:

$$C=48.90\pm2.0\%; \quad H=7.05\pm2.0\%; \quad N=14.80\pm2.0\%$$

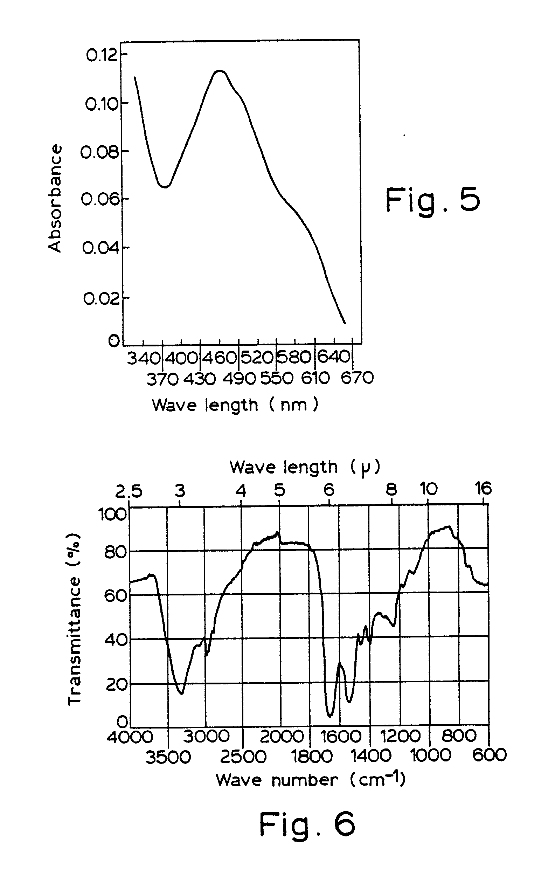

(i) Absorptions in the ultraviolet and visible regions of the spectrum: Fig. 4 shows an ultraviolet absorption spectrum of this enzyme and Fig. 5 shows a visible absorption spectrum of this enzyme.

In the UV region, this enzyme gives an absorption maximum at about 280 nm, an absorption minimum at about 253 nm and a shoulder at about 290 nm. The absorbance ratio (260 nm/280 nm) is 1.8. In the visible region, the enzyme shows an absorption maximum at about 470 to 475 nm, an absorption minimum at 375 to 380 nm and a shoulder at about 610 nm.

(j) Infrared absorption spectrum: Fig. 6 shows an infrared absorption spectrum of this enzyme measured by the KBr method. Characteristic absorptions are 3.02 (s), 3.38 (m), 6.00 (s), 6.60 (s), 6.95 (m), 7.25 (m), 8.10 (m), 8.50 (w), 9.10 (w).

(k) Molar extinction coefficient: The molar extinction coefficient of the enzyme at 280 nm is about $1.08 \times 10^5 \ M^{-1} \cdot cm^{-1}$.

(l) Amino acid analysis: The enzyme is hydrolyzed with 6N-HCl at 110°C for a predetermined time period (24—72 hrs.) and analyzed for amino acids by means of an amino acid analyzer. The values given in the following table denote the number of amino acid residues per molecule of the enzyme (mol. wt.=$4.8 \times 10^4$).

| Amino acid | Number of residues | Amino acid | Number of residues | Amino acid | Number of residues |
|---|---|---|---|---|---|
| Lysine | 28.8 | Tryptophan | 14.4 | Serine | 20.2 |
| Histidine | 13.9 | Aspartic acid | 47.0 | Glutamic acid | 40.3 |
| Arginine | 10.6 | Threonine | 18.7 | Proline | 13.9 |
| Glycine | 23.5 | Valine | 22.6 | Leucine | 28.3 |
| Alanine | 48.4 | Methionine | 5.3 | Tyrosine | 18.2 |
| Cysteine | 0 | Isoleucine | 10.6 | Phenylalanine | 19.3 |

(m) Analysis for metal: As determined by the atomic absorption method, this enzyme contains about 1.5 gram-atom of manganese. It is, therefore, clear that the enzyme is a Mn-superoxide dismutase.

The enzymatic potency assay procedure is as follows.

①The method described in the Journal of Biological Chemistry *244*, 6049—6055 (1969) is employed. Thus, a sample cell (length of light path 1 cm) is filled with 1.5 ml of 100 mM phosphate buffer (pH 7.8), 0.5

4

ml of 60 µM cytochrome C, 0.5 ml of 0.6 mM ethylenediaminetetracetate sodium, 0.25 ml of 0.6 mM xanthine and 0.21 ml of the enzyme solution diluted with phosphate buffer (pH 7.8) to a measurable concentration, followed by addition of 0.04 ml of xanthine oxidase solution to make a total of 3.0 ml. The cell is set on a spectrophotometer and the reaction is initiated at 25°C so as to measure the initial velocity (v) of reduction of cytochrome C at 550 nm. As a blank, the initial velocity (V) of reduction of cytochrome C in the presence of distilled water in lieu of the enzyme solution is also measured. With the amount of the enzyme causing a 50% inhibition of the initial velocity of reduction of cytochrome C under the above reaction conditions being taken as unity, the enzymatic potency is calculated by means of the following equation.

$$U/mg = \frac{(V)-(v)}{(v)\times amount\ of\ enzyme\ (mg)/1.0\ ml\ reaction\ mixture}$$

②The active dye method described in Analytical Biochemistry *44*, 276—287, 1971 can also be utilized. thus, after polyacrylamide gel electrophoresis, the gel is immersed in 2.45 mM nitro blue tetrazolium for 20 minutes. Then, the gel is further immersed in a mixture of 28 mM tetraethylethylenediamine, 0.028 mM riboflavine and 36 mM potassium phosphate (pH 7.8) for 15 minutes. The gel is put in a test tube and exposed to the light rays of a 15-watt fluorescent lamp, whereupon the superoxide ion $O_2^-$ generated by photoreduction of riboflavine reduces the nitro blue tetrazolium so that the moiety other than that having superoxide dismutase activity forms a blue-formazan and is therefore dyed blue-purplish.

Proteins can be assayed by the method of Lowry [The Journal of Biological Chemistry *193*, 265—275, 1951].

The superoxide dismutase according to this invention differs in physicochemical properties from the known superoxide dismutase. The known superoxide dismutase includes the one obtained from *Escherichia coli* B [The Journal of Biological Chemistry Vol. 245, No. 22, pp. 6176—6181, 1970] and the one derived from *Bacillus stearothermophilus* [Journal of Molecular Biology, vol. 105, pp. 333—335, 1976]. The molecular weight of the superoxide dismutase derived from *Escherichia coli* is 39,500 and that of the superoxide dismutase from *Bacillus stearothermophilus* is 40,000. Since these weight figures are significantly different from the molecular weight of the enzyme of this invention which is about $4.8 \times 10^4$, the superoxide dismutase according to this invention is considered to be a novel enzyme.

The superoxide dismutase of the present invention shows an overt inhibition of edema when a sample of 3000 U/mg is administered intravenously into the hind leg of the rat according to the carrageenan-induced foot edema method [Van Arman. C. G. et al: The Journal of Pharmacology and Experimental Therapeutics vol. 150, p. 328 (1965)] at the dose of at least 1.0 mg/kg.

Effect to nystatin edema

To a paw of JCL-ICR mouse is subcutaneously injected 0.025 ml of 1% nystatin solution in physiological saline. After six hours from said administration, edema is emerged, and then superoxide dismutase (SOD) (11,000 U/mg · protein) of the present invention is subcutaneously injected to the portion.

This experiment is conducted according to the method described in Pharmacology vol. 5, pp. 215—224 (1971). The results are set forth in the following table.

| Treatment | Dose U/paw | No. of mice | Edema volume (mg) mean±S.E. | Inhibition (%) |
|---|---|---|---|---|
| Control | — | 10 | 120.45±8.87 | — |
| SOD | 50 | 10 | 92.35±4.38 | 23.3 |
| SOD | 500 | 10 | 83.71±3.28 | 30.5 |

S.E.=standard error

As is clear from said results, superoxide dismutase of the present invention shows a tendency of inhibiting nystatin edema.

The enzyme of this invention is sparingly toxic and no death is encountered even when it is intraperitoneally administered to rats at a dose of 60 mg/kg.

Having an antiinflammatory activity, the superoxide dismutase according to this invention is of use as an antiinflammatory agent.

For the treatment of various acute or subacute inflammatory diseases or edema (e.g. arthritis, bronchitis, edema due to a bruise, burn, etc.) in mammalian animals (e.g. mouse, rat, rabbit, cat, dog, monkey, man), the enzyme according to this invention can be administered orally or otherwise as formulated into tablets, capsule, injections, ointments or other known dosage forms at a daily dose of 1 to 5 mg/kg.

Examples of the pharmaceutically acceptable carrier or excipient are albumin, globulin, dextran, Ficoll Type 70 (Sigma Co., U.S.A.), lactose, dextrin and starch.

The following examples are given to illustrate this invention in further detail without limiting its scope. Unless otherwise specified, percents (%) are weight/volume percents (w/v %).

Example 1

A 200 ml-conical flask was filled with 40 ml of a liquid medium containing 1.0% of solvent-extracted soybean meal, 1.5% of casein, 0.6% of diammonium phosphate, 0.1% of sodium chloride, 0.05% of potassium chloride, 0.02% of calcium chloride, 0.02% of magnesium sulfate, 1.0% of calcium carbonate, 0.0021% of zinc carbonate, 0.6% of soybean oil and 0.1% of Actocol® (adjusted to pH 7.0), and was sterilized with steam at 120°C for 20 minutes. The flask was inoculated with a loopful of one of the undermentioned strain of the genus *Serratia* and incubated at 28°C on a rotary shaker revolving at 230 r.p.m. for 48 hours. The fermentation broth was centrifuged to separate the cells which were lyophilized at −20°C. A 10-gram portion of the cellular lyophilizate was taken and reconstituted at 30°C. Following addition of 40 ml of 20 mM phosphate buffer (pH 7.8), the cells were disrupted by ultrasonication (2A, 5 minutes). The disrupted cells were centrifuged to give a supernatant fluid. This supernatant was measured for superoxide dismutase activity by the above-described xanthine-xanthine oxidase-cytochrome C method and polyacrylamide gel electrophoresis method. The results are set forth below in the table. It is clear that in all strains of *Serratia*, strong superoxide dismutase was intracellularly accumulated.

| Strain | Deposit No. | Activity (U/mg · protein) |
|---|---|---|
| *Serratia liquefaciens* | IFO 12979 | 74 |
| *Serratia marcescens* | IFO 3046 | 67 |
| *Serratia marcescens* | IFO 3759 | 115 |
| *Serratia marcescens* | IFO 3736 | 91 |
| *Serratia marinorubra* | IFO 12973 | 200 |

Example 2

(a) Harvesting the cells

A 2-liter Sakaguchi flask was filled with 500 ml of a liquid medium of the same composition as described in Example 1 and, after sterilization, inoculated with *Serratia marcescens* IFO 3736. The flask was incubated at 28°C on a rotary shaker revolving at 230 r.p.m. for 24 hours to give a seed culture. A 200-liter tank was charged with 100 l of a medium of the same composition as above and under sparging at the rate of 100 l/min., submerged aerobic culture was conducted at 28°C. The resulting fermentation broth was centrifuged to separate the cells which were lyophilized at −20°C.

(b) Preparing a crude enzyme product

A 1.5-kg portion of the wetted cell was reconstituted at 30°C and after addition of 3 l of 20 mM phosphate buffer (pH 7.8), the cells were disrupted in a Dynomill (Willy A. Bachofen Manufacturing Engineers, Switzerland) apparatus and centrifuged in a Sharples type centrifuge to give a clear supernatant. From this supernatant, the precipitate obtained with 30—70% (W/V) of ammonium sulfate was collected by centrifugation. This sediment was dissolved in 20 mM phosphate buffer containing a small amount of 0.1 M KCl (pH 7.0) and, in a cellulose tube, dialyzed against the same type of buffer for 3 days. The internal fluid, i.e. 150 ml of dialysate, was concentrated to 30 ml by ultrafiltration through a Diaflow UM-5 membrane [an ultrafiltration membrane of Amicon Far East Limited, U.S.A.]. The concentrate was loaded onto a column (4.0×66.0 cm) of Sephadex G-150® previously equilibrated with the same buffer solution and eluted with the same buffer to collect 210 ml of an enzymatically active fraction. To this fraction was added ammonium sulfate to a concentration of 70% (W/V) and the resultant precipitate was collected by centrifugation and dissolved in a small amount of water. In a cellulose tube, this solution was dialyzed against water for 3 days and the internal fluid was lyophilized to give 620 mg of a crude enzyme product. The specific activity of this product was 3600 U/mg · protein.

(c) Preparing a pure enzyme product
(i) Column chromatography on diethylaminoethyl-cellulose

In a small amount of 20 mM tris-hydrochloric acid buffer (pH 9.0) was dissolved 620 mg of the crude enzyme obtained in (b), and in a cellulose tube, the solution was dialyzed against the same buffer for 3 days. The internal fluid, i.e. 80 ml of dialysate, was put on a column (2.5×45.0 cm) of diethylaminoethyl-cellulose previously equilibrated with the same buffer and the enzyme was eluted by increasing the concentration of sodium chloride in the same buffer linearly from 0 to 250 mM to collect 45 ml of an enzymatically active eluate.

(ii) Gel filtration on Sephadex G-100®

The enzyme solution obtained in (i) (45 ml) was put in a cellulose tube and dialyzed against 20 mM phosphate buffer containing 0.1 M of KCl. The internal fluid (60 ml) was concentrated to 6.0 ml by ultrafiltration through a Dyaflow UM-5 membrane. This concentrate was put on a column (2.6×74.0 cm) of Sephadex G-100® equilibrated with the same buffer as above and the enzyme was eluted with the same buffer to collect an enzymatically active eluate which was red-purple in color. The fractions thus obtained are constant in specific activity, and polyacrylamide gel electrophoresis shows that it is a single component product. The specific activity of this Mn-superoxide dismutase (24 mg) was 12,000 U/mg · protein.

Example 3

*Serratia marcescens* IFO 3759 was treated in the manner as Example 2 to give 1.5 kg of lyophilized cells. and, then, 410 mg of crude enzyme therefrom. The specific activity of this enzyme product was 3630 U/mg · protein. This enzyme was further purified by the procedure as Example 2 to give a pure Mn-superoxide dismutase product. The specific activity of this pure product was 12,000 U/mg · protein.

Example 4

*Serratia marinorubra* IFO 12973 was treated in the same manner as Example 2 to give 1.5 kg of lyophilized cells and, thence, 830 mg of crude enzyme product. The specific activity of this product was 3,800 U/mg · protein.

Example 5
(a) Harvesting the cells

A 2000 ml of Sakaguchi flask was filled with 500 ml of a liquid medium containing 1.0% of solvent-extracted soybean meal, 1.5% of casein, 0.6% of diammonium phosphate, 0.1% of sodium chloride, 0.05% of potassium chloride, 0.02% of calcium chloride, 0.02% of magnesium sulfate, 1.0% of calcium carbonate, 0.0021% of zinc carbonate, 0.6% of soybean oil and 0.1% of Actocol® (adjusted to pH 7.0), and, after sterilization, inoculated with *Serratia marcescens* ATCC 21074. The flask was incubated at 28°C on a rotary shaker revolving at 230 r.p.m. for 24 hours to give a seed culture. A 200-liter tank was charged with 100 l of a medium of the same composition as above and under sparging at the rate of 100 l/min., submerged aerobic culture was conducted at 28°C. The resulting fermentation broth was centrifuged to separate the cells which were lyophilized at −20°C.

(b) Preparing a crude enzyme product

A 5-kg portion of the wetted cell was reconstituted at 30°C and after addition of 10 l of 20 mM phosphate buffer (pH 7.8), the cells were disrupted in a Dynomill apparatus and centrifuged in a Sharples type centrifuge to give a clear supernatant. From this supernatant, the precipitate obtained with 30—70% (W/V) of ammonium sulfate was collected by centrifugation. This sediment was dissolved in 20 mM phosphate buffer containing a small amount of 0.1 M KCl (pH 7.0) and, in a cellulose tube, dialyzed against the same type of buffer for 3 days. The internal fluid, i.e. 530 ml of dialysate, was concentrated to 40 ml by ultrafiltration through a Diaflow-UM-5 membrane. The concentrate was loaded onto a column (4.0×66.0 cm) of Sephadex G-150® previously equilibrated with the same buffer solution and eluted with the same buffer to collect 550 ml of an enzymatically active fraction. To this fraction was added ammonium sulfate to a concentration of 70% (W/V) and the resultant precipitate was collected by centrifugation and dissolved in a small amount of water. In a cellulose tube, this solution was dialyzed against water for 3 days and the internal fluid was lyophilized to give 2070 mg of a crude enzyme product. The specific activity of this product was 3600 U/mg · protein.

(c) Preparing a pure enzyme product
(i) Column chromatography on diethylaminoethyl-cellulose

In a small amount of 20 mM tris-HCl buffer (pH 9.0) was dissolved 2070 mg of the crude enzyme obtained in (b), and in a cellulose tube, the solution was dialyzed against the same buffer for 3 days. The internal fluid, i.e. 134 ml of dialysate, was put on a column (3.8×55.0 cm) of diethylaminoethyl-cellulose previously equilibrated with the same buffer and the enzyme was eluted by increasing the concentration of sodium chloride in the same buffer linearly from 0 to 250 mM to collect 54 ml of an enzymatically active eluate.

(ii) Gel permeation on Sephadex G-100®

The enzyme solution obtained in (i) (54 ml) was put in a cellulose tube and dialyzed against 20 mM phosphate buffer containing 0.1 M of KCl. The internal fluid (73 ml) was concentrated to 2.0 ml by ultrafiltration through a Dyaflow UM-5 membrane. This concentrate was put on a column (2.6×74.0 cm) of

7

**0 045 222**

Sephadex G-100® equilibrated with the same buffer as above and the enzyme was eluted with the same buffer to collect an enzymatically active eluate which was red-purple in color. The fractions thus obtained are constant in specific activity, and polyacrylamide gel electrophoresis shows that it is a single component product. The specific activity of this Mn-superoxide dismutase (2.07 mg) was 12,000 U/mg · protein.

The mode of preparation of the Mn-superoxide dismutase which is described above employing *Serratia marcescens* ATCC 21074 has been described in our Application EPA 70656 (82 303600.9) (designating Belgium, Federal Republic of Germany, France, Italy, Netherlands, Sweden, Siwtzerland and the United Kingdom) with a priority based on U.S. Application 285316 of the 22nd July 1981, and only forms part of the present application so far as the designated state of Austria is concerned.

Example of the preparation

(1) An enteric capsule is obtained by *per se* conventional manner employing the following ingredients:

| | |
|---|---|
| Superoxide dismutase of the present invention (3,600 U/mg) | 10 mg |
| Lactose | 46 mg |
| Corn starch | 16 mg |
| Crystalline cellulose | 12 mg |
| Cellulose glycollic acid | 5 |
| Sorbitol | 10 mg |
| | 99 mg (per capsule) |

(2) An enteric tablet is obtained by *per se* conventional manner employing the following ingredients:

| | |
|---|---|
| Superoxide dismutase of the present invention (3,600 U/mg) | 10 mg |
| Lactose | 79 mg |
| Corn starch | 45.5 mg |
| Magnesium stearate | 0.5 mg |
| | 135 mg (per tablet) |

Said two capsules or tablets are usually administered for adult human after meal, and the administration is carried out three times a day.

**Claims for the Contracting States: BE, CH, DE, FR, GB, IT, LI, LU, NL, SE**

1. A Mn-superoxide dismutase having the following properties:

(a) Activity: to dismutase a superoxide ion into a hydrogen peroxide molecule and an oxygen molecule;

(b) Substrate specificity: to act on superoxide ion;

(c) Optimum pH range: about 7 to 8;

(d) Stable pH range: about 6 to 11;

(e) Optimum functional temperature range: about 20 to 45°C;

(f) Thermostability: Stable at 37°C for 60 minutes;
  Inactivated about 50% at 50—60°C in 60 minutes;
  Inactivated about 100% at 80°C in 5 minutes;

(g) Molecular weight; about $4.8 \times 10^4$ (by gel filtration method);

(h) Elemental analysis: C 48.90±2.0%;
  H 7.05±2.0%;
  N 14.80±2.0%;

(i) Absorptions in the visible region of the spectrum: 470—475 nm (max.), 375—380 nm (min.), about 610 nm (shoulder);

(j) Molar extinction coefficient: about $1.08 \times 10^5 M^{-1} \cdot cm^{-1}$ at 280 nm;

(k) Amino acid analysis

| Amino acid | Number of residues |
|---|---|
| Glycine | 23.5 |
| Leucine | 28.3 |

(The values denotes the number of amino acid residues per molecule of the enzyme).

8

2. Method of producing a Mn-superoxide dismutase according to claim 1 characterised by cultivating a Mn-superoxide dismutase-producing microorganism belonging to the genus *Serratia* (excluding Serratia marcescens ATCC 21074) in a culture medium to thereby cause the microorganism to elaborate and accumulate said Mn-superoxide dismutase and harvesting said Mn-superoxide dismutase from the resulting culture broth.

3. A method as claimed in Claim 2, wherein the microorganism belongs to the species *Serratia liquefaciens, Serratia marcescens* or *Serratia marinorubra*.

4. A method as claimed in Claim 2, wherein the microorganism is *Serratia liquefaciens* IFO 12979, *Serratia marcescens* IFO 3046, *Serratia marcescens* IFO 3759, *Serratia marcescens* IFO 3736, or *Serratia marinorubra* IFO 12973.

(5) An antiinflammatory agent which contains an effective amount of a Mn-superoxide dismutase having the following properties:

(a) Activity: to dismutase a superoxide ion into a hydrogen peroxide molecule and an oxygen molecule;

(b) Substrate specificity: to act on superoxide ion;

(c) Optimum pH range: about 7 to 8;

(d) Stable pH range: about 6 to 11;

(e) Optimum functional temperature range: about 20 to 45°C;

(f) Thermostability: Stable at 37°C for 60 minutes;

    Inactivated about 50% at 50—60°C in 60 minutes;

    Inactivated about 100% at 80°C in 5 minutes;

(g) Molecular weight: about $4.8 \times 10^4$ (by gel filtration method);

(h) Elemental analysis: C $48.90 \pm 2.0\%$;

                 H $7.05 \pm 2.0\%$;

                 N $14.80 \pm 2.0\%$;

(i) Absorptions in the visible region of the spectrum: 470—475 nm (max.), 375—380 nm (min.), about 610 nm (shoulder);

(j) Molecular extinction coefficient: approx. $1.08 \times 10^5 M^{-1} \cdot cm^{-1}$ at 280 nm;

(k) Amino acid analysis:

| Amino acid | Number of residues |
| --- | --- |
| Glycine | 23.5 |
| Leucine | 28.3 |

(The values denote the number of amino acid residues per molecule of the enzyme), as an active ingredient in association with a pharmaceutically acceptable carrier or excipient therefor.

6. A Mn-superoxide dismutase, having the following properties:

(a) Activity: to dismutate a superoxide ion into a hydrogen epoxide molecule and an oxygen molecule;

(b) Substrate specificity: to act on superoxide ion;

(c) Optimum pH range: about 7 to 8;

(d) Stable pH range: about 6 to 11;

(e) Optimum functional temperature range: about 20 to 45°C;

(f) Thermostability: stable at 37°C for 60 minutes;

    Inactivated about 50% at 50—60°C in 60 minutes;

    Inactivated about 100% at 80°C in 5 minutes;

(g) Molecular weight: about $4.8 \times 10^4$ (by gel filtration method);

(h) Elemental analysis: C $48.90 \pm 2.0\%$;

                 H $7.05 \pm 2.0\%$;

                 N $14.80 \pm 2.0\%$;

(i) Absorptions in the visible region of the spectrum: 470—475 nm (max.), 375—380 nm (min.), about 610 nm (shoulder);

(j) Molar extinction coefficient: about $1.08 \times 10^5 M^{-1} \cdot cm$ at 280 nm;

(k) Amino acid analysis:

| Amino acid | Number of residues |
| --- | --- |
| Glycine | 23.5 |
| Leucine | 28.3 |

(The values denotes the number of amino acid residues per molecule of the enzyme), for therapeutic use as an antiinflammatory agent.

# 0 045 222

**Claims for the Contracting State: AT**

1. A method of producing a Mn-superoxide dismutase using the following properties:

(a) Activity: to dismutase a superoxide ion into hydrogen peroxide molecule and an oxygen molecule;

(b) Substrate specificity: to act on superoxide ion;

(c) Optimum pH range: about 7 to 8;

(d) Stable pH range: about 6 to 11;

(e) Optimum functional temperature range: about 20 to 45°C;

(f) Thermostability: Stable at 37°C for 60 minutes;
Inactivated about 50% at 50—60°C in 60 minutes;
Inactivated about 100% at 80°C in 5 minutes;

(g) Molecular weight: about $4.8 \times 10^4$ (by gel filtration method);

(h) Elemental analysis: C $48.90 \pm 2.0\%$;
H $7.05 \pm 2.0\%$;
N $14.80 \pm 2.0\%$;

(i) Absorptions in the visible region of the spectrum: 470—475 nm (max.), 375—380 nm (min.), about 610 nm (shoulder);

(j) Molecular extinction coefficient: about $1.08 \times 10^5 M^{-1} \cdot cm^{-1}$ at 280 nm;

(k) Amino acid analysis:

| Amino acid | Number of residues |
|---|---|
| Glycine | 23.5 |
| Leucine | 28.3 |

(The values denotes the number of amino acid residues per molecule of the enzyme), characterised by cultivating a Mn-superoxide dismutase-producing microorganism belonging to the genus *Serratia* in a culture medium to thereby cause the microorganism to elaborate and accumulate said Mn-superoxide dismutase, and harvesting said Mn-superoxide dismutase from the resulting culture broth.

2. A method as claimed in Claim 1 wherein the microorganism belongs to the species *Serratia liquefaciens, Serratia marcescens,* or *Serratia marinorubra.*

3. A method as claimed in Claim 2, wherein the microorganism is *Serratia liquefaciens* IFO 12979, *Serratia marcescens* IFO 3046, *Serratia marcescens* IFO 3759, *Serratia marcescens* IFO 3736, *Serratia marcescens* ATCC 21074 or *Serratia marinorubra* IFO 12973.

**Patentansprüche für die Vertragsstaaten: BE, CH, DE, FR, GB, IT, LI, LU, NL & SE**

1. Mn-Superoxid-Dismutase mit den folgenden Eingenschaften:

(a) Aktivität: zur Dismutierung eines Superoxid-Ions in ein Hydrogenperoxid-Molekül und einer Sauerstoff-Molekül;

(b) Substrat-Spezifität: zur Wirkung auf ein Superoxid-Ion;

(c) optimaler pH-Bereich: etwa 7 bis 8;

(d) stabiler pH-Bereich: etwa 6 bis 11;

(e) optimaler funktioneller Temperatur-Bereich: etwa 20°C bis 45°C;

(f) Thermostabilität: 60 min stabil bei 37°C; inaktiviert in 60 min bei 50—60°C etwa 50%, inaktiviert in 5 min bei 80°C etwa 100%;

(g) Molekulargewicht: etwa $4,8 \times 10^4$ (mittels der Gel-Filtrationsmethode);

(h) Elementaranalyse: C $48,90 \pm 2,0\%$;
H $7,05 \pm 2,0\%$;
N $14,80 \pm 2,0\%$;

(i) Absorptionen im Sichtbaren Bereich des Spektrums: 470—475 nm (max.), 375—380 nm (min.), etwa 610 nm (Schulter);

(j) Molarer Extinktionskoeffizient: bei 280 nm etwa $1,08 \times 10^5$ mol$^{-1} \cdot 1 \cdot cm^{-1}$;

(k) Aminosäure-Analyse:

| Aminosäure | Zahl der Reste |
|---|---|
| Glycin | 23,5 |
| Leucin | 28,3 |

(Die Werte bezeichnen die Zahlen der Aminosäure-Reste pro Molekül des Enzyms).

2. Verfahren zur Herstellung einer Mn-Superoxid-Dismutase nach Anspruch 1, dadurch gekennzeichnet, daß ein Mn-Superoxid-Dismutase erzeugender Mikroorganismus, der zu der Gattung Serratia (ausschließlich Serratia marcescens ATCC 21074) gehört, in einem Kulturmedium kultiviert wird, wodurch der Mikroorganismus zur Bildung und Anreicherung der Mn-Superoxid-Dismutase veranlaßt wird, und die Mn-Superoxid-Dismutase aus der resultierenden Kulturbrühe geerntet wird.

3. Verfahren nach Anspruch 2, dadurch gekennzeichnet, daß der Mikroorganismus zu den Species Serratia liquefaciens, Serratia marcescens oder Serratia marinorubra gehört.

4. Verfahren nach Anspruch 2, dadurch gekennzeichnet, daß der Mikroorganismus Serratia liquefaciens IFO 12979, Serratia marcescens IFO 3046, Serratia marcescens IFO 3759, Serratia marcescens IFO 3736 oder Serratia marinorubra IFO 12973 ist.

5. Antiinflammatorisches Mittel, enthaltend eine wirksame Menge einer Mn-Superoxid-Dismutase mit den folgenden Eigenschaften:

(a) Aktivität: zur Dismutierung eines Superoxid-Ions in ein Hydrogenperoxid-Molekül und ein Sauerstoff-Molekül;

(b) Substrat-Spezifität: zur Wirkung auf ein Superoxid-Ion;

(c) optimaler pH-Bereich: etwa 7 bis 8;

(d) stabiler pH-Bereich: etwa 6 bis 11;

(e) optimaler funktioneller Temperatur-Bereich: etwa 20°C bis 45°C;

(f) Thermostabilität: 60 min stabil bei 37°C; inaktiviert in 60 min bei 50—60°C etwa 50%; inaktiviert in 5 min bei 80°C etwa 100%;

(g) Molekulargewicht: etwa $4,8 \times 10^4$ (mittels der Gel-Filtrationsmethode);

(h) Elementaranalyse: C 48,90±2,0%;

                    H   7,05±2,0%;

                    N 14,80±2,0%;

(i) Absorptionen im sichtbaren Bereich des Spektrums: 470—475 nm (max.), 375—380 nm (min.), etwa 610 nm (Schulter);

(j) Molarer Extinktionskoeffizient: bei 280 nm etwa $1,08 \times 10^5$ $mol^{-1} \cdot 1 \cdot cm^{-1}$;

(k) Aminosäure-Analyse:

| Aminosäure | Zahl der Reste |
| --- | --- |
| Glycin | 23,5 |
| Leucin | 28,3 |

(Die Werte bezeichnen die Zahlen der Aminosäure-Reste pro Molekül des Enzyms), als wirkstoff in Verbindung mit einem pharmazeutisch annehmbaren Träger oder Streckmittel für diesen.

6. Mn-Superoxid-Dismutase mit den folgenden Eigenschaften:

(a) Aktivität: zur Dismutierung eines Superoxid-Ions in ein Hydrogenperoxid-Molekül und ein Saurestoff-Molekül;

(b) Substrat-Spezifität: zur Wirkung auf ein Superoxid-Ion;

(c) optimaler pH-Bereich: etwa 7 bis 8;

(d) stabiler pH-Bereich: etwa 6 bis 11;

(e) optimaler funktioneller Temperatur-Bereich: etwa 20°C bis 45°C;

(f) Thermostabilität: 60 min stabil bei 37°C; inaktiviert in 60 min bei 50—60°C etwa 50%, inaktiviert in 5 min bei 80°C etwa 100%;

(g) Molekulargewicht: etwa $4,8 \times 10^{-4}$ (mittels der Gel-Filtrationsmethode);

(h) Elementaranalyse: C 48,90±2,0%;

                    H   7,05±2,0%;

                    N 14,80±2,0%;

(i) Absorptionen im sichtbaren Bereich des Spektrums: 470—475 nm (max.), 375—380 nm (min.), etwa 610 nm (Schulter);

(j) Molarer Extinktionskoeffizient: bei 280 nm etwa $1,08 \times 10^5$ $mol^{-1} \cdot 1 \cdot cm^{-1}$;

(k) Aminosäure-Analyse:

| Aminosäure | Zahl der Reste |
| --- | --- |
| Glycin | 23,5 |
| Leucin | 28,3 |

(Die Werte bezeichnen die Zahlen der Aminosäure-Reste pro Molekül des Enzyms), zur therapeutischen Verwendung als antiinflammatorisches Mittel.

**Patentansprüche für den Vertragsstaat: AT**

1. Verfahren zur Erzeugung einer Mn-Superoxid-Dismutase mit den folgenden Eigenschaften:

(a) Aktivität: zur Dismutierung eines Superoxid-Ions in ein Hydrogenperoxid-Molekül und ein Saurestoff-Molekül;

(b) Substrat-Spezifität: zur Wirkung auf ein Superoxid-Ion;

(c) optimaler pH-Bereich: etwa 7 bis 8;

(d) stabiler pH-Bereich; etwa 6 bis 11;

(e) optimaler funktioneller Temperatur-Bereich: etwa 20°C bis 45°C;

(f) Thermostabilität: 60 min stabil bei 37°C; inaktiviert in 60 min bei 50—60°C etwa 50%; inaktiviert in 5 min bei 80°C etwa 100%;

(g) Molekulargewicht: etwa $4,8 \times 10^4$ (mittels der Gel-Filtrationsmethode);

(h) Elementaranalyse: C $48,90 \pm 2,0\%$;

H $7,05 \pm 2,0\%$;

N $14,80 \pm 2,0\%$;

(i) Absorptionen im sichtbaren Bereich des Spektrums: 470—475 nm (max.), 375—380 nm (min.), etwa 610 nm (Schulter);

(j) Molarer Extinktionskoeffizient: bei 280 nm etwa $1,08 \times 10^5$ $mol^{-1} \cdot 1 \cdot cm^{-1}$;

(k) Aminosäure-Analyse:

| Aminosäure | Zahl der Reste |
|---|---|
| Glycin | 23,5 |
| Leucin | 28,3 |

(Die Werte bezeichnen die Zahlen der Aminosäure-Reste pro Molekül des Enzyms), dadurch gekennzeichnet, daß ein Mn-Superoxid-Dismutase erzeugender Mikroorganismus, der zu der Gattung Serratia gehört, in einem Kulturmedium kultiviert wird, wodurch der Mikroorganismus zur Bildung und Anreicherung der Mn-Superoxid-Dismutase veranlaßt wird, und die Mn-Superoxid-Dismutase aus der resultierenden Kulturbrühe geerntet wird.

2. Verfahren nach Anspruch 1, dadurch gekennzeichnet, daß der Mikroorganismus zu den Species Serratia liquefaciens, Serratia marcescens oder Serratia marinorubra gehört.

3. Verfahren nach Anspruch 2, dadurch gekennzeichnet, daß der Mikroorganismus Serratia liquefaciens IFO 12979, Serratia marcescens IFO 3046, Serratia marcescens IFO 3759, Serratia marcescens IFO 3736, Serratia marcescens ATCC 21074 oder Serratia marinorubra IFO 12973 ist.

**Revendications pour les Etats Contractants: BE, CH, DE, FR, GB, IT, LI, LU, NL et SE**

1. Superoxyde dismutase Mn, qui présente les propriétés suivantes:

(a) Activité: la dismutation d'un ion superoxyde en une molécule de peroxyde d'hydrogène et une molécule d'oxygène;

(b) Spécificité du substrat: l'action sur l'ion superoxyde;

(c) Domaine optimum de pH: environ 7 à 8;

(d) Domaine stable de pH: environ 6 à 11;

(e) Domaine optimum de température fonctionnelle: environ 20 à 45°C;

(f) Stabilité thermique: stable à 37°C pendant 60 minutes; Inactivée à environ 50% à 50—60°C en 60 minutes; Inactivée à environ 100% à 80°C en 5 minutes;

(g) Poids moléculaire: environ $4,8 \times 10^4$ (par la méthode de filtration sur gel);

(h) Analyse élémentaire: C $48,90 \pm 2,0\%$;

H $7,05 \pm 2,0\%$;

N $14,80 \pm 2,0\%$;

(i) Absorptions dans la région visible du spectre: 470—475 nm (max.), 375—380 nm (min.), environ 610 nm (épaule);

(j) Coefficient d'extinction moléculaire: environ $1,08 \times 10^5$ $M^{-1} \cdot cm^{-1}$ à 280 nm;

(k) Analyse des acides aminés:

| Acide aminé | Nombre de restes |
|---|---|
| Glycine | 23,5 |
| Leucine | 28,3 |

(Les valeurs indiquent le nombre de restes d'acide aminé par molécule de l'enzyme).

2. Procédé de préparation de superoxyde dismutase Mn selon la revendication 1, caractérisé en ce que l'on cultive un microorgane producteur de superoxyde dismutase Mn, appartenant au genre Serratia (à l'exception de Serratia marcescens ATCC 21704) dans un milieu de culture, de façon à faire produire et accumuler par le microorganisme ladite superoxyde dismutase Mn, et l'on récolte ladite superoxyde dismutase Mn à partir du bouillon de culture résultant.

3. Procédé selon la revendication 2, dans lequel le microorganisme appartient à l'espèce *Serratia liquefaciens*, *Serratia marcescens* ou *Serratia marinorubra*.

4. Procédé selon la revendication 2, dans lequel le microorganisme est *Serratia liquefaciens* IFO 12979, *Serratia marcescens* IFO 3046, *Serratia marcescens* IFO 3759, *Serratia marcescens* IFO 3736 ou *Serratia marinorubra* IFO 12973.

5. Agent anti-inflammatoire qui contient une quantité efficace de superoxyde dismutase Mn ayant les propriétés suivantes:

(a) Activité: la dismutation d'un ion superoxyde en une molécule de peroxyde d'hydrogène et une molécule d'oxygène;

(b) Spécificité du substrat: l'action sur l'ion superoxyde;

(c) Domaine optimum de pH: environ 7 à 8;

(d) Domaine stable de pH; environ 6 à 11;

(e) Domaine optimum de température fonctionnelle: environ 20 à 45°C;

(f) Stabilité thermique: stable à 37°C pendant 60 minutes; Inactivée à environ 50% à 50—60°C en 60 minutes; Inactivée à environ 100% à 80°C en 5 minutes;

(g) Poids moléculaire: environ $4,8\times10^4$ (par la méthode de filtration sur gel);

(h) Analyse élémentaire: C $48,90\pm2,0\%$;
  H $7,05\pm2,0\%$;
  N $14,80\pm2,0\%$;

(i) Absorptions dans la région visible du spectre: 470—475 nm (max.), 375—380 nm (min.), environ 610 nm (épaule);

(j) Coefficient d'extinction moléculaire: environ $1,08\times10^5$ $M^{-1}\cdot cm^{-1}$ à 280 nm;

(k) Analyse des acides aminés:

| Acide aminé | Nombre de restes |
| --- | --- |
| Glycine | 23,5 |
| Leucine | 28,3 |

(Les valeurs indiquent le nombre de restes d'acides aminé par molécule de l'enzyme); en tant que substance active, en association avec une matière de support ou un excipient pharmaceutiquement acceptable.

6. Superoxyde dismutase Mn, qui présente les propriétés suivantes:

(a) Activité: la dismutation d'un ion superoxyde en une molécule de peroxyde d'hydrogène et une molécule d'oxygène;

(b) Spécificité du substrat: l'action sur l'ion superoxyde;

(c) Domaine optimum de pH: environ 7 à 8;

(d) Domaine stable de pH: environ 6 à 11;

(e) Domaine optimum de température fonctionnelle: environ 20 à 45°C;

(f) Stabilité thermique: stable à 37°C pendant 60 minutes; Inactivée à environ 50% à 50—60°C en 60 minutes; Inactivée à environ 100% à 80°C en 5 minutes;

(g) Poids moléculaire: environ $4,8\times10^4$ (par la méthode de filtration sur gel);

(h) Analyse élémentaire: C $48,90\pm2,0\%$;
  H $7,05\pm2,0\%$;
  N $14,80\pm2,0\%$;

(i) Absorptions dans la région visible du spectre: 470—475 nm (max.), 375—380 nm (min.), environ 610 nm (épaule);

(j) Coefficient d'extinction moléculaire: environ $1,08\times10^5$ $M^{-1}\cdot cm^{-1}$ à 280 nm;

(k) Analyse des acides aminés:

| Acide aminé | Nombre de restes |
| --- | --- |
| Glycine | 23,5 |
| Leucine | 28,3 |

(Les valeurs indiquent le nombre de restes d'acide aminé par molécule de l'enzyme); pour son utilisation thérapeutique comme agent anti-inflammatoire.

**Revendications pour l'Etat Contractant: AT**

1. Procédé de préparation de superoxyde dismutase Mn, qui présente les propriétés suivantes:

(a) Activité: la dismutation d'un ion superoxyde en une molécule de peroxyde d'hydrogène et une molécule d'oxygène;

13

**0 045 222**

(b) Spécificité du substrat: l'action sur l'ion superoxyde;
(c) Domaine optimum de pH: environ 7 à 8;
(d) Domaine stable de pH: environ 6 à 11;
(e) Domaine optimum de température fonctionelle: environ 20 à 45°C;
(f) Stabilité thermique: stable à 37°C pendant 60 minutes; Inactivée à environ 50% à 50—60°C en 60 minutes; Inactivée à environ 100% à 80°C en 5 minutes;
(g) Poids moléculaire: environ $4,8 \times 10^4$ (par la méthode de filtration sur gel);
(h) Analyse élémentaire: C 48,90±2,0%;
$\quad$ H 7,05±2,0%;
$\quad$ N 14,80±2,0%;
(i) Absorptions dans la région visible du spectre: 470—475 nm (max.), 375—380 nm (min.), environ 610 nm (épaule);
(j) Coefficient d'extinction moléculaire: environ $1,08 \times 10^5$ $M^{-1} \cdot cm^{-1}$ à 280 nm;
(k) Analyse des acides aminés:

| Acide aminé | Nombre de restes |
|---|---|
| Glycine | 23,5 |
| Leucine | 28,3 |

(Les valeurs indiquent le nombre de restes d'acide aminé par molécule de l'enzyme); caractérisé en ce que l'on cultive un microorgane producteur de superoxyde dismutase Mn, appartenant au genre *Serratia* dans un milieu de culture, de façon à faire produire et accumuler par le microorgansme ladite superoxyde dismutase Mn, et l'on récolte ladite superoxyde dismutase Mn à partir du bouillon de culture résultant.

2. Procédé selon la revendication 1, dans lequel le microorganisme appartient à l'espèce *Serratia liquefaciens*, *Serratia marcescens* ou *Serratia marinorubra*.

3. Procédé selon la revendication 2, dans lequel le microorganisme est *Serratia liquefaciens* IFO 12979, *Serratia marcescens* IFO 3046, *Serratia marcescens* IFO 3759, *Serratia marcescens* IFO 3736, *Serratia marcescens* ATCC 21074 ou *Serratia marinorubra* IFO 12973.

14

0 045 222

Fig.1

—o—Acetate buffer solution

—●—Phosphate buffer solution

—✕—Glycine sodium hydroxide buffer
solution

Fig. 2

1

0 045 222

Fig. 3

Fig. 4

2

Fig. 5

Fig. 6